Europäisches Patentamt

European Patent Office

Office européen des brevets

(11) Publication number: **0 308 162**
**A2**

# EUROPEAN PATENT APPLICATION

(21) Application number: **88308416.2**

(22) Date of filing: **12.09.88**

(51) Int. Cl.⁴: **A61B 5/10 , G06K 9/00**

(30) Priority: **15.09.87 US 97591**

(43) Date of publication of application:
**22.03.89 Bulletin 89/12**

(84) Designated Contracting States:
**AT BE CH DE ES FR GB GR IT LI LU NL SE**

(71) Applicant: **IDENTIX INCORPORATED**
**510 North Pastoria**
**Sunnyvale California 94086(US)**

(72) Inventor: **Sartor, Thomas F.**
**1603 Lawrence Street**
**Eugene Oregon, 97401(US)**
Inventor: **Driscoll, Edward C., Jr.**
**11 Sandstone**
**Portola Valley California 94025(US)**

(74) Representative: **Ayers, Martyn Lewis Stanley**
**et al**
**J.A. KEMP & CO. 14 South Square Gray's Inn**
**London, WC1R 5EU(GB)**

(54) Optical system for fingerprint imaging.

(57) An optical system for fingerprint imaging utilizing a platen prism and a correction prism. The platen prism includes a platen area on one face for receiving a finger to be imaged. Light from a monochromatic light source enters the platen prism through a second face and is totally internally reflected at the platen area surface except in the regions where ridges of the finger directly contact the platen prism. The correction prism receives the light from the platen prism. An objective lens receives the light from the correction prism and forms a fingerprint image on a light detector. The geometry and placement of the correction prism, with respect to the platen prism, are selected to reduce defocussing of the fingerprint image on the detector introduced by the tilting of the platen area with respect to the axis of the objective lens. The light detector, typically an area array detector such as a charge coupled device array, provides an output used for fingerprint verification and the like. Preferably, the correction prism, in combination with the platen prism, also provides anamorphic magnification of the fingerprint image thereby permitting the aspect ratio of the image to be controlled. Thus, the image aspect ratio can be selected to match the aspect ratio of the light sensitive cells which form the area array detector.

EP 0 308 162 A2

FIG. 2.

## OPTICAL SYSTEM FOR FINGERPRINT IMAGING

Background of the Invention

Optical systems have been developed for the purpose of optically recording an image of a fingerprint. One type of system includes a stationary transparent arcuate optical platen for receiving a finger and a moving light source which scans the optical platen so as to generate a fingerprint image. U.S.P. No. 4,537,484 entitled "Fingerprint Imaging Apparatus" discloses such a mechanical scanning arrangement. A second type of system typically utilizes an optical platen in the form of a prism. A finger to be imaged is placed on one surface of the platen prism and the prism is internally illuminated. Light is then scattered at those locations on the prism surface where the ridges of the fingerprint make direct contact with the surface. A typical optical system utilizing a platen prism is disclosed in U.S.P. No. 3,527,535 entitled "Fingerprint Observation and Recording Apparatus".

One advantage of the second type of optical system over the first type is that there are no moving mechanical parts. However, it is more difficult to acquire a sufficiently large fingerprint image from a platen having a planar surface as opposed to an arcuate surface. In order to obtain a fingerprint image utilizing a planar platen of sufficient quality for fingerprint verification and the like, it has been necessary to utilize a relatively complex optical system. Such systems are inherently high in cost and cannot easily be implemented in compact form.

The present invention overcomes the above-noted shortcomings of conventional optical systems for fingerprint imaging. The fingerprint is imaged without the use of a mechanical scanner. Moreover, the image is of sufficient quality for use in fingerprint verification applications without the need for complex and high cost optical elements. Furthermore, the subject system can be readily configured in a compact package. These and other advantages of the subject invention will become apparent to those skilled in the art upon a reading of the Description of the Preferred Embodiment together with the drawings.

Summary of the Disclosure

An optical system for fingerprint imaging is disclosed. The system includes a platen prism having a platen area on the face thereof for receiving a finger to be imaged. The platen prism is internally illuminated through another face by a light source, such as a light emitting diode, with light from the source striking the platen area at an angle of incidence sufficiently large to cause the light to be totally internally reflected.

When a finger is positioned on the platen area, internal reflection is frustrated in those areas where the ridges of the finger make direct contact with the surface of the prism. Thus, the light reflected at the platen surface will form a fingerprint image.

The reflected light exits the platen prism through a third prism face. The axial principal ray of the reflected fingerprint image is tilted with respect to the prism surface so as to defocus the image near the image edges. A correction prism is provided which includes a pair of faces which form a correction angle. Light from the platen prism enters the correction prism through one face and exits through the second face.

The system further includes a light detector such as a charge coupled device (CCD) array. An objective lens is provided for receiving light from the correction prism and for forming a fingerprint image on the light detector. The objective lens defines an optical axis, an entrance pupil and an optical plane which is normal to the optical axis and positioned at the entrance of the objective lens.

The correction prism, in combination with the platen prism, function to equalize the effective optical path lengths of the principal rays originating from different points on the platen area of the platen prism to the optical plane of the objective lens. This may be accomplished by selecting a correction prism angle and the relative positions of the two prisms so as to reduce the difference in effective optical path lengths. Preferably, the difference in effective optical path lengths from opposite edges of the platen area to the objective lens optical plane are reduced to less than 5% of the average optical path length. Thus, defocussing created by the tilting of the fingerprint image exiting the platen prism is reduced.

In a preferred embodiment, the correction prism angle and relative position of the two prisms are also selected to form an image on the light detector having a predetermined desired aspect ratio. The correction prism functions to provide anamorphic magnification (magnification along a single axis) so as to vary the image aspect ratio. In the event an array detector comprised of an array of light sensitive cells is utilized, the aspect ratio of the image can be adjusted to match the cell aspect ratio so that the fingerprint image will be uniformly sampled by the array detector.

## Brief Description of the Drawings

Figure 1 is a perspective view of the subject optical system with a portion of the housing cut away to show the arrangement of the internal optical elements.

Figure 2 is a top plan view of the subject optical system, with the top of the housing cut away.

Figure 3 is a side elevational view of the subject optical system, with a side of the housing cut away.

Figure 4 is a schematic diagram of the optical system, with the image folding mirrors and light source deleted.

Figure 5 is a simplified block diagram of the electronic circuitry associated with the subject optical system.

## Description of the Preferred Embodiment

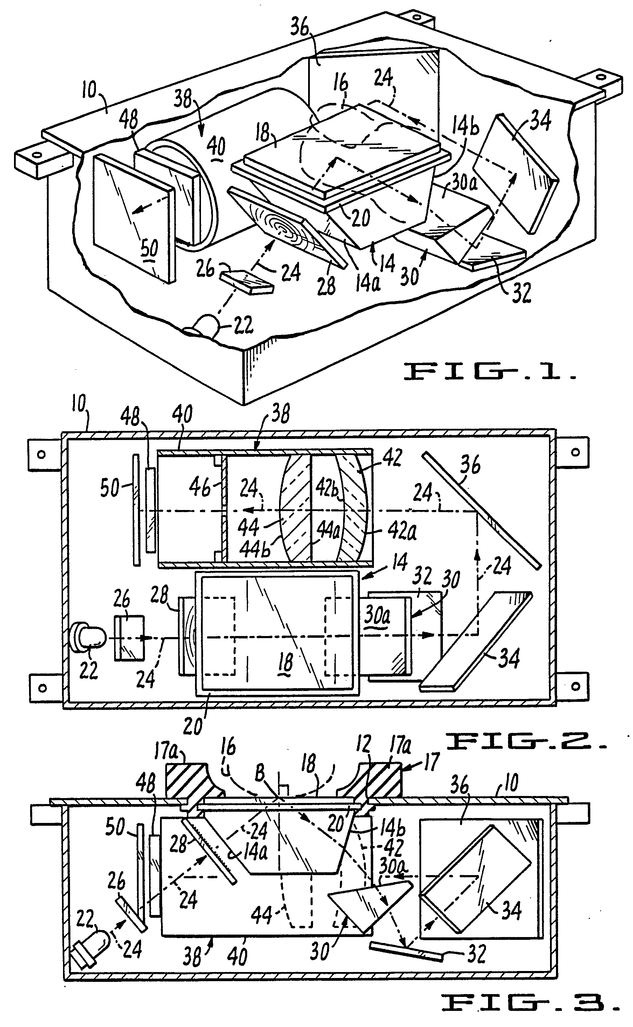

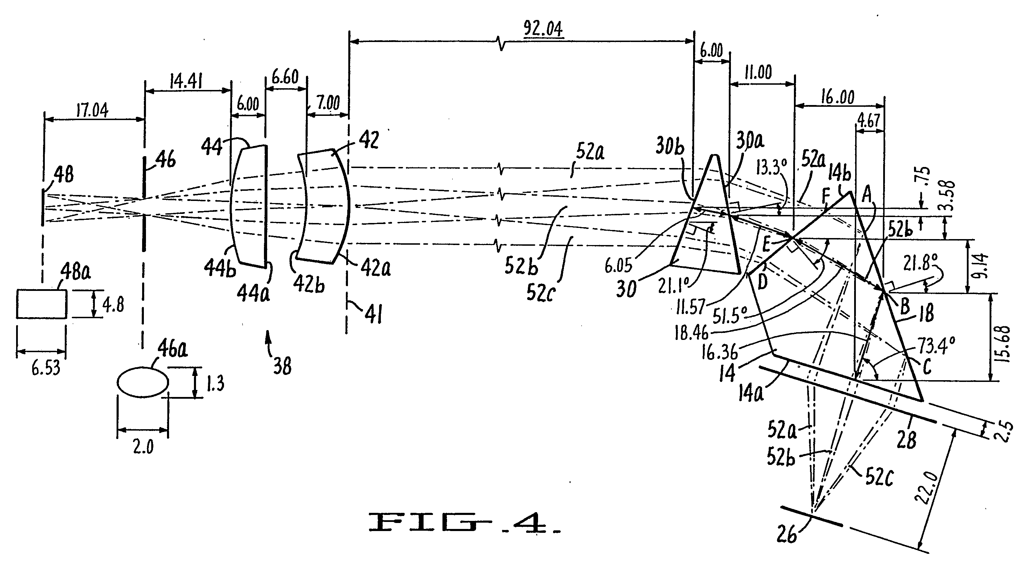

Referring now to Figures 1-3 of the drawings, a preferred embodiment of the subject optical system for fingerprint imaging may be seen. Many conventional features, well known in the art, such as to the structure for mounting the various components which comprise the subject optical system have been deleted so as not to obscure the true nature of the invention in unnecessary detail.

The components which make up the system are mounted in housing 10. The system includes a source of diffused monochromatic light. A Light Emitting Diode (LED) 22 generates red monochromatic light, with the axial principal ray of the light represented by line 24. The wave length of the light in the presently-preferred embodiment is 660 nm ± 10 nm. A GaAlAs LED with a typical radiant intensity of 3000 mcd along the central axis has been found suitable for the present application. One such LED is marketed by Toshiba under the designation TLRA150.

An optical diffuser 26 is positioned in front of LED 22. Diffuser 26, which is preferably opal glass, receives an evenly illuminated spot of light having a diameter of about 3 mm.

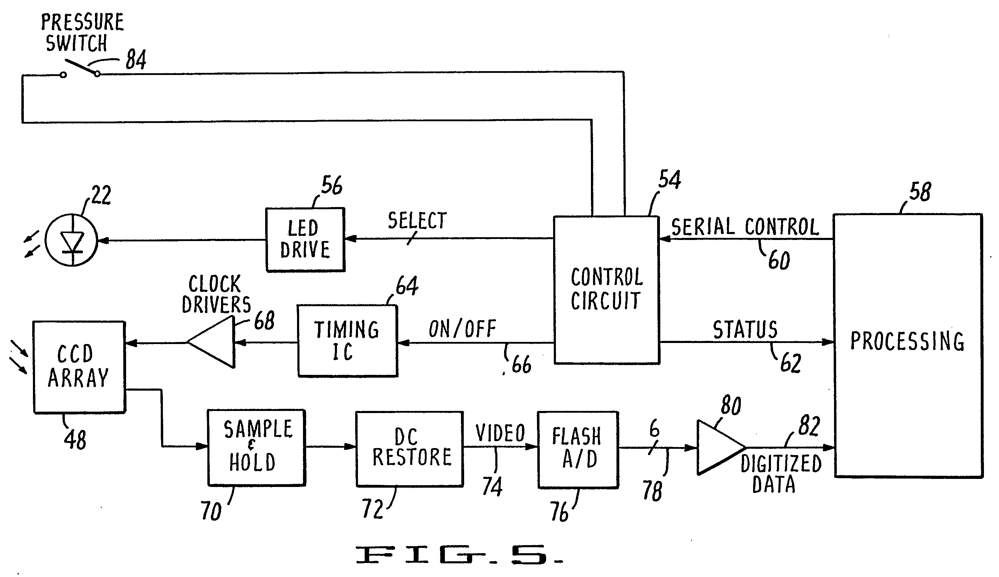

A collimating lens 28 is positioned along the optical axis for collimating the diffused light provided by diffuser 26. The relative spacing of the optical components in the presently-preferred embodiment of the system are set forth in the Figure 4 schematic diagram. Lens 28, which can be a low-cost plastic fresnel lens, has a focal length of 22 mm and is positioned such that diffuser 26 is located in the focal plane of the lens. Accordingly, the rays through the center of the illuminated spot on diffuser 26 are approximately parallel when the rays exit lens 28. The beam of collimated light produced by lens 28 has a diameter of 25 mm.

A platen prism, generally designated by the numeral 14, is located along the optical axis of the collimated light. Platen prism 14 is a trapezoidal prism, preferably made of acrylic plastic, which is mounted in a rectangular opening 12 in housing 10. Plastic is preferably over glass since glass has been found to be more likely to retain latent fingerprints. Prism 14 includes a peripheral flange 20 for mounting purposes. A rubber grommet 17 is utilized for securing prism 14 in the housing.

As can best be seen in Figure 3, grommet 17 includes an external peripheral notch (not designated) for engaging the lip of housing 10 and an internal peripheral notch (not designated) for receiving flange 20. Grommet 17 defines a platen area on platen surface 18 for receiving a finger 16 to be imaged. Grommet 17 includes raised members 17a extending away from housing 10 which function as guides for the finger.

As shown in Figure 3, platen prism 14 has an entrance face 14a which is normal to principal ray 24. Entrance face 14a forms an angle of 38.5 degrees with the horizontal platen surface 18 at the top of the prism. The rays strike platen surface 18 at an angle of incidence of 51.5 degrees, which is well above the critical angle of 42.2 degrees for an air/acrylic interface. Accordingly, total internal reflection of the rays occur at surface 18.

When a finger 16 is placed on the platen area of surface 18, the ridges of the finger directly contact the platen surface. Such contact causes those rays striking the ridges to be frustrated, thereby preventing internal reflection. Those segments of finger 16 intermediate the ridges do not contact the platen surface, therefore light in the areas below the segments will still be reflected. As a result, the light which is reflected at the surface 18 under the platen area forms an image of the fingerprint. Point B (Figure 3) represents the

3

location on the platen area at which principal ray 24 is reflected.

The light reflected at surface 18 exits prism 14 through exit face 14b. exit face 14b defines an angle of 73.3 degrees with respect to platen surface 18 of the prism. The platen area on surface 18 imaged by the subject system is 19 mm square.

Grommet 17 permits platen prism 14 to be slightly displaced downward when finger pressure is applied to the platen surface 18. A pressure-actuated switch (not depicted) senses finger pressure and causes the imaging process to be initiated. Grommet 17 also permits prism 14 to be easily replaced.

The light reflected at the platen area of surface 18 exits prism 14 at an angle of 39.8 degrees with respect to the normal of surface 18. Thus, the fingerprint image formed at surface 18 will appear tilted. As will be subsequently described in greater detail, a correction prism, generally designated by the numeral 30, is provided for removing the image defocus created by the tilting of the fingerprint image. Correction prism 30 also provide magnification of the fingerprint image along one axis so that the finger will be uniformly sampled by the light detector.

The objective lens of the system, generally designated by the numeral 38, is located within housing 10, adjacent platen prism 14. The optical axis of lens 38 is parallel to the optical path formed by lens 28 and prisms 14 and 30. In order to minimize the size of the disclosed optical system, the objective lens of the system is positioned adjacent platen prism 14. The optical axis of lens 38 is offset from the optical path formed by lens 28. Accordingly, the rays exiting correction prism 30 must be folded around to permit objective lens 38 to be mounted as shown.

A set of three mirrors 32, 34 and 36 are used for folding the rays so as to bend the optical path 180 degrees and to offset the optical path. Mirrors 32, 34 and 36 are preferably front surface aluminized mirrors. First mirror 32 (Fig. 3) reflects the rays exiting correction prism 30 at an angle of 45 degrees with respect to platen surface 18. The plane of reflection is the same as that of the incident rays. Second mirror 34 is mounted such that the rays are then reflected orthogonally from the plane of rays from exit face 30b of prism 30. Finally, the third mirror 76 reflects the rays parallel to platen surface 18 and parallel to the plane of rays from exit face 30b. Preferably, second mirror 34 is adjustable so as to permit correction of slight errors in the positioning of the remaining optical elements of the system

Objective lens 38 includes a cylindrical housing 40 co-axial with principal ray 24 reflected by mirror 36. The lens includes a pair of lens elements 42 and 44 and an aperture stop 46. Objective lens 38 provides a magnification of 0.33.

A Charge Coupled Device (CCD) area array 48 is positioned to receive light from objective lens 38. A low cost CCD array having 384 x 490 light sensitive cells which are at a spacing of 17.0μm x 10.0μm may be used for this application. A CCD array meeting these specifications is marketed by Sanyo under the designation LC9921.

As previously noted, correction prism 30 functions to remove image defocus created because the axial principal ray 24 of the image is tilted with respect to the platen area on surface 18. Accordingly, the effective optical path lengths of the principal rays of light reflected at different locations on surface 18 to the objective lens will not be the same. The term principal ray refers to light rays leaving a particular point on the platen area of surface 18 which passes through the center of the entrance pupil (or the aperture of stop 46) of objective lens 38. The axial principal ray 24 is the principal ray leaving the center of the platen area (point B) on surface 18. The axial principal ray 24 aligns with the optical axis of lens 38 and terminates at the center of the fingerprint image formed on CCD array 48. The term effective optical path length, as used herein, is the total length of the optical path normalized to air. By way of example, the section of the path through prism 14 is divided by the index of refraction of the prism (1.49) so as to normalize that section to air.

The foregoing can be further illustrated by reference to the schematic diagram of the subject optical system in Figure 4. The light source (LED 22) and folding mirrors 23, 24 and 36 are not depicted. The various linear dimension are in millimeters (mm) and are referenced to either the horizontal or vertical axes of the figure unless otherwise depicted. The angles of the respective prism surfaces are referenced to the normal of the prism surface and the horizontal axis.

The light from diffuser 28 is shown broken down into three bundles of light rays for purposes of illustration. The bundles include marginal bundles 52a and 52c and axial bundle 52b. Bundles 52a and 52c strike opposite edges of the platen area on surface 18 designated as points A and C, respectfully. Axial bundle 52b strikes the center of the platen area designated as point B.

Since the angle of incidence of bundles 52a, 52b and 52c is greater than the critical angle, the light will be totally internally reflected unless frustrated by a fingerprint ridge. The reflected light at surface 18 which forms the fingerprint image exits the prism at points D, E and F on face 14b. Thus, the fingerprint image viewed at face 14b will be tilted with respect to platen surface 18.

In the absence of correction prism 30, the fingerprint image exiting prism 14 will be defocussed at the edges because the image is tilted. The resultant difference in effective optical path lengths of light bundles 52a, 52b, 52c to the objective lens will be negligible provided the lens is located a substantial distance from the prism. However, in order to reduce the size of the system, it is necessary to place the objective lens relatively close to prism 14. In that event, the difference in optical path lengths in comparison to the average path length will be large and substantial defocus will result.

One function of correction prism 30 is to reduce the difference in effective optical path lengths of the rays which form the fingerprint image. The light path of concern begins at surface 18 of prism 14 and ends at an optical plane 41 located at objective lens 38. Optical plane 41 is normal to the optical axis of the lens and positioned at the entrance point of the lens.

The correction prism angle defined by the respective faces 30a and 30b of correction prism 30, together with the orientation of the correction prism with respect to platen prism 14, are selected so as to reduce the effective optical path lengths of the light which form the fingerprint image. Preferably, the difference in effective optical path lengths of the light emanating from opposite edges A and C of the platen area on surface 18 to optical plane 41 of objective lens 38 is less than 5 percent of the average effective optical path length.

A second function of correction prism 30 is to provide anamorphic (magnification along a single axis) magnification of the fingerprint image. The axis of magnification is normal to the line of intersection of the planes which lie on prism faces 30a and 30b. Such magnification ensures that the fingerprint image at surface 18 is uniformly sampled by CCD array 48.

The light sensitive cells of most low cost CCD arrays have a non-uniform spacing. As previously noted, CCD 48 has a cell spacing of 10$\mu$m by 17$\mu$m with such spacing providing a cell aspect ratio of 0.59 (10$\mu$m/17$\mu$m). The sample spacing of the platen area on which the fingerprint image is formed is preferably square. In the presently-preferred embodiment, the sampling spacing of the fingerprint is selected at 50.2$\mu$m by 50.2$\mu\mu$, which corresponds to a unity aspect ratio. The platen area is also square and measures 19mm by 19mm. Thus, the image aspect ratio is unity.

As also previously noted, it is desirable that the fingerprint image formed at the platen area be uniformly sampled by the CCD array. Otherwise, the resolution of the image will be dependent upon the orientation of the finger when it is placed on the platen. Correction prism 30 functions to provide magnification along a single axis so as to modify the aspect ratio of the fingerprint image so that the ratio matches the cell aspect ratio of the CCD array. If the two ratios are matched, the fingerprint image will be uniformly sampled by the array, even though the array cells have an aspect ratio other than unity (non-uniform pitch).

The correction prism angle together with the orientation of the correction prism with respect to the platen prism, can be selected to provide the desired anamorphic magnification. In the presently-preferred embodiment, the angle and position are selected to provide a magnification of 0.59. Thus, the size of the image between edges A and C on the finger receiving platen area is reduced by a factor of 0.59. The same angle and orientation simultaneously provide the desired defocussing correction by equalizing the effective optical path lengths. A correction prism angle of 34.4 degrees, with entry face 30a positioned at an angle of 8.5 degrees with respect to surface 18 of prism 14, provide the desired defocussing correction and anamorphic magnification for the Figure 4 system.

Correction prism 30 permits the aspect ratio of the fingerprint image to be selected to match that of the cells aspect ratio of the CCD array 48. The objective lens 38 magnification is selected so that the fingerprint image formed by the objective lens on the array matches the dimensions of the light sensitive cells of the array. An objective lens magnification of 0.33, along with the anamorphic magnification of 0.59, for the system depicted in Figure 4 reduces the desired sampling size of the finger platen area of 50.2$\mu$m by 50.2$\mu$m to correspond to the array at spacings at 17.0$\mu$m by 10.0$\mu$m.

The use of prisms with broad-spectrum light, such as from an incandescent lamp, causes severe chromatic aberrations. Since the light provided by LED 22 is monochromatic, these aberrations are avoided. The previously mentioned device selected as LED 22 has a very tight output beam. The diffuser 26 spreads the beam into an even illumination over the condenser lens 28. The use of lens 28 makes efficient use of the light from LED 22 by refracting the light from the illuminated spot on the diffuser parallel to the principal rays through the fingerprint image. This causes a much greater proportion of the light from LED 22 to contribute to the fingerprint image than if the image were illuminated by diffuse light from all angles.

In addition to the defocussing of the fingerprint image on the detector 48 due to the tilt of the platen area on surface 28 with respect to the axial principal ray 24 at the surface, there is distortion caused at the edges of the image if the principal rays for the edges are not parallel to the axial principal ray. The distortion may be eliminated by positioning the objective lens a long distance from the prisms, such that the principal rays through the lens 38 are all roughly parallel. Such an approach would increase the size of the system. It is preferable to design objective lens 38 with an entrance pupil at a far distance from the prisms, while keeping the lens near the prisms. The entrance pupil of lens 38 is defined as the image of aperture stop 46 as viewed from the object side of the lens. Such an entrance pupil is referred to herein as a remote entrance pupil because the pupil is located to the image side of objective lens 38 and is outside the lens. The entrance pupil is specified for lens 38 at 130 mm behind the front surface of the lens. This limits the difference in angle for the principal rays for the marginal bundles 52a and 52c from the axial principal ray 52c at 4 degrees, which adequately reduces the distortion. In most applications, the angle should be less than 10 degrees. A currently preferred objective lens 38 is specified in Table 1.

Table 1

| Object Lens Specification | |
|---|---|
| Pupil Effective Entrance | 12.5mm x 19.2mm @ 250 mm |
| Magnification | 0.334 |
| Clear Front Aperture Diameter | 20.7mm |
| Front Vertex To Image Spacing | 120mm |
| Design Wavelength | 660nm (no color correction) |

Lens elements 42 and 44 of objective lens 38 are preferably plastic aspheric elements. The parameters for the four surface 42a, 42b, 44a and 44b of lens elements 42 and 44 are set forth in Table 2.

Table 2

| Surface | Radius | Conicity |
|---|---|---|
| 42a | 14.57mm | -0.582 |
| 42b | 26.87mm | 0 |
| 44a | flat | 0 |
| 44b | -24.38mm | -6.360 |

The high effective f/number of lens 38 relaxes the tolerances on focusing, aberrations and distortion. The aperture of stop 46 is narrower in the plane of refraction through prisms 14 and 30. Aberrations related to the diameter of a light bundle at a surface are worse in the axis where refraction occurs than in the axis where the surface is normal to the light. The dimensions of the aperture of stop 46 are 1.3mm x 2.0mm. This results in an effective f/number for the lens 38 of f/20 x f/13.

The exact dimensions and designs of the system were obtained with the use of an optical design program which optimized the lens prescriptions and the angles and placements of the prism to obtain an optimum compromise between distortion and blur size of the fingerprint image while minimizing the size and complexity of the elements. Thus, the embodiment was configured according to the reasons provided previously, but the final selection of element prescriptions were based on optimization of all factors on the basis of simulated measurements of distortion and blur size. Embodiments with differences in the weight put open the simulation measurements to be optimized results in different prescriptions for the elements although the configuration will stay similar.

Referring now to Figure 5, a block diagram of the control apparatus for use with the subject optical system is depicted. The components which make up the control apparatus are preferably mounted on one or more circuit boards which are located within housing 10. A suitably-programmed microprocessor 58 operates in conjunction with a control unit 54 for performing an imaging operation. The operation is commenced when pressure sensitive switch 84 is actuated by depression of platen prism 14. Control circuit 54 notifies process 58, as represented by status line 62. Processor 58 responds by causing the control circuit to energize LED 22 by way of drive circuit 56.

Control circuit 54 also turns on CCD timing circuit 64 which provides timing signals for the CCD array through drive 68. A timing circuit marketed by Sanyo under the designation LC9902 is suitable for this application. The CCD array is scanned at a standard 30 frames/second which determines the integration time for each frame.

The analog video data out of the CCD array is transferred to a sample and hold circuit 70. The sample and hold circuit contains a voltage which corresponds to the light level of an individual CCD cell and represents what is sometimes referred to as a pixel (picture element). D.C. restore circuit 72 functions to provide an offset voltage which sets the black reference level when the masked (dark) cells of the CCD array are scanned.

The video signal from the D.C. restore circuit is digitized by a flash A/D converter. The digitized data for each pixel are then forwarded to the processing unit 58 by way of driver 80. Unit 58 then stores the video data or processes the data further for the propose of comparing the fingerprint image data with stored data for identification, verification and the like.

Thus, an optical system for use in a fingerprint imaging apparatus has been disclosed. Although a preferred embodiment of the system has been described in some detail, it is understood that obvious variations can be made without departing from the spirit and scope of the invention as defined by the appended claims.

## Claims

1. An optical system for fingerprint imaging comprising:
light means for producing a source of light;
a platen prism having first, second and third faces, with said second face having a rectangular-shaped platen area for receiving a finger to be imaged, with said area having first and second opposite edges generally parallel to the line of intersection of the planes defined by said second and third faces, and with said light means and said platen prism being positioned relative to one another such that light from said light means enters said platen prism through said first face, strikes said second face at an angle of incidence greater than a critical angle determined by the respective indices of refraction of said prism and a medium adjacent said second face so that light is internally reflected at said second face which exits said platen prism through said third face, with light reflected from said platen area on said second face forming a fingerprint image when a finger is positioned on said platen area;
an objective lens having an optical axis, an optical plane at an entrance of said objective lens normal to said optical axis and an entrance pupil;
light detector means for detecting light provided by said objective lens;
a correction prism, intermediate said platen prism and said objective lens, having fourth and fifth faces which define a correction prism angle therebetween, with said correction prism angle and the relative positions of said correction prism and said platen prism being selected such that light from said third face of said platen prism enters said correction prism through said fourth face and exits from said fifth face and is received by said objective lens and further selected so that a difference in effective optical path lengths of principal rays of light reflected at said first edge of said platen area and said second edge of said platen area to said objective lens optical plane is reduced.

2. The optical system of Claim 1 wherein said difference in effective optical path lengths is reduced to less than 5% of the average path length.

3. The optical system of Claim 2 wherein said correction prism angle and said relative positions of said correction prism and said platen prism are also selected to produce anamorphic magnification of said fingerprint image along a first axis normal to said first and second edges of said platen area.

4. The optical system of Claim 3 wherein said light detector means includes an area array detector comprised of light sensitive cells, with said cells arranged with respect to one another so as to have a predetermined cell aspect ratio and wherein said anamorphic magnification is selected such that said fingerprint image has an image aspect ratio at said optical plane which matches said call aspect ratio.

5. The optical system of Claim 4 wherein said cell aspect ratio is other than unity.

6. The otpical system of Claim 4 wherein said area array detector is a charge coupled device array.

7. The optical system of Claim 4 further including an aperture stop intermediate said objective lens and said area array detector.

8. The optical system of Claim 4 wherein said aperture stop includes an asymmetrical aperture with the height of said aperture along said first axis of anamorphic magnification being less than the width of said aperture.

9. The optical system of Claim 2 wherein said light produced by said light means is substantially monochromatic light.

10. The optical system of Claim 9 wherein said light means includes a light emitting diode.

11. The otpical system of Claim 10 wherein said light means inlcudes a condenser lens intermediate said light emitting diode and said platen prism.

12. The optical system of Claim 11 wherein said light means includes an optical diffuser disposed between said light emitting diode and said condenser lens.

13. The optical system of Claim 12 wherein said condenser lens is a fresnel lens and light existing said fresnel lens is collimated light.

14. The optical system of Claim 13 wherein said fresnel lens, said platen prism and said correction prism are plastic.

15. The optical system of Claim 2 wherein said objective lens entrance pupil is a remote entrance pupil.

16. The optical system of Claim 15 wherein the distance between said remote entrance pupil and the said fifth face is sufficient to reduce an angle between said principal rays from said first and second edges with respect to an axial principal ray from a point on said platen area equidistant from said first and second edges to less than 10 degrees.

17. An optical system for fingerprint imaging comprising:

light means for producing a source of light;

a platen prism having one face that includes a platen area for receiving a finger to be imaged, with said light means and said platen prism being positioned relative to one another so that light enters said platen prism and is internally reflected at said platen area on said one face so as to produce a fingerprint image when a finger is positioned on said platen area;

a correction prism positioned for receiving said fingerprint image directly from said platen prism;

an area array light detector; and

an objective lens positioned so as to receive light from said correction prism and to form said fingerprint image on said area array light detector..

18. The optical system of Claim 17 wherein said light means includes a light emitting diode which provides substantially monochromatic light.

19. The optical system of Claim 18 wherein said light means includes a fresnel lens and a light diffuser intermediate said fresnel lens and said light emitting diode.

20. The optical system of Claim 19 wherein said fresnel lens, said platen prism and said correction prism are plastic.

21. The optical system of Claim 17 wherein said correction prism includes a pair of faces which form a correction prism angle, wherein said correction prism angle and the relative positions of said platen prism and said correction prism are selected such that defocussing of said fingerprint image formed on said area array detector is reduced.

22. The optical system of Claim 21 wherein said area array detector includes an array of light sensitive cells arranged with respect to one another to provide a predetermined cell aspect ratio and wherein said correction prism angle and said relative position of said platen prism and said correction prism are further selected such that said fingerprint image formed on said array has an aspect ratio which matches said cell aspect ratio.

**FIG.1.**

**FIG.2.**

**FIG.3.**

FIG.4.

EP 0 308 162 A2

FIG.5.

PRESSURE SWITCH 84

22

LED DRIVE 56

SELECT

CONTROL CIRCUIT 54

SERIAL CONTROL 60

PROCESSING 58

CLOCK DRIVERS 68

TIMING IC 64

ON/OFF 66

STATUS 62

CCD ARRAY 48

SAMPLE & HOLD 70

DC RESTORE 72

VIDEO 74

FLASH A/D 76

6 78

80

DIGITIZED DATA 82

EP 0 308 162 A2